(19) **Europäisches Patentamt / European Patent Office / Office européen des brevets**

(11) **EP 3 053 601 B1**

(12) **EUROPEAN PATENT SPECIFICATION**

(45) Date of publication and mention
of the grant of the patent:
**08.11.2017 Bulletin 2017/45**

(21) Application number: **14851311.2**

(22) Date of filing: **30.09.2014**

(51) Int Cl.:
*A61K 48/00* (2006.01)    *A61K 38/17* (2006.01)
*A61K 38/16* (2006.01)    *A61P 35/00* (2006.01)
*C12N 9/02* (2006.01)    *A61K 38/44* (2006.01)

(86) International application number:
**PCT/KR2014/009204**

(87) International publication number:
**WO 2015/050364 (09.04.2015 Gazette 2015/14)**

(54) **PHARMACEUTICAL COMPOSITION FOR PREVENTING AND TREATING CANCER, CONTAINING CYB5R3 GENE OR PROTEIN AS ACTIVE INGREDIENT**

PHARMAZEUTISCHE ZUSAMMENSETZUNG ZUR VORBEUGUNG UND BEHANDLUNG VON KREBS MIT CYB5R3-GEN ODER PROTEIN ALS WIRKSTOFF

COMPOSITION PHARMACEUTIQUE POUR PRÉVENIR ET TRAITER UN CANCER, CONTENANT LE GÈNE OU LA PROTÉINE CYB5R3 EN TANT QUE SUBSTANCE ACTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2013 KR 20130117527**

(43) Date of publication of application:
**10.08.2016 Bulletin 2016/32**

(73) Proprietor: **Korea Research Institute of Bioscience and Biotechnology**
**Daejeon 305-806 (KR)**

(72) Inventors:
• **WON, Mi Sun**
**Daejeon 305-806 (KR)**
• **BAN, Hyun Seung**
**Daejeon 305-806 (KR)**
• **KIM, Bo Kyung**
**Daejeon 305-806 (KR)**
• **NAM, Soon Woo**
**Seoul 138-788 (KR)**
• **LEE, Kyeong**
**Seoul 100-715 (KR)**
• **LEE, Young-Ju**
**Daejeon 305-806 (KR)**
• **LEE, Hong-Sub**
**Hwaseong-si**
**Gyeonggi-do 445-170 (KR)**

• **KIM, Dong Uk**
**Daejeon 305-806 (KR)**
• **PARK, Ki Cheol**
**Daejeon 305-720 (KR)**
• **OH, Kyoung-Jin**
**Daejeon 34098 (KR)**
• **IM, Joo-Young**
**Yuseong-gu, Daejeon 34140 (KR)**

(74) Representative: **Office Freylinger**
**P.O. Box 48**
**8001 Strassen (LU)**

(56) References cited:
**CN-A- 1 303 926        KR-A- 20080 042 162**
**KR-A- 20120 002 613    KR-B1- 100 387 452**
**US-A1- 2006 204 503**

• YOON: "Profiling of transcripts and proteins modulated by K-ras oncogene in the lung tissues of K-ras transgenic mice by omics approaches", INTERNATIONAL JOURNAL OF ONCOLOGY, 1 January 1992 (1992-01-01), XP055276796, GR ISSN: 1019-6439, DOI: 10.3892/ijo_00000138
• A MARÍN ET AL: "DT-diaphorase and cytochrome B5 reductase in human lung and breast tumours", BRITISH JOURNAL OF CANCER, vol. 76, no. 7, 1 October 1997 (1997-10-01), pages 923-929, XP055276903, GB ISSN: 0007-0920, DOI: 10.1038/bjc.1997.485

**(Cont. next page)**

Remarks:
    The file contains technical information submitted after the application was filed and not included in this specification

Remarks:
    The file contains technical information submitted after the application was filed and not included in this specification

**Description**

**BACKGROUND OF THE INVENTION**

**1. Field of the Invention**

The present invention relates to a pharmaceutical composition for use in preventing and treating certain cancers, containing cytochrome b5 reductase 3 (cyb5R3) protein as an active ingredient.

**2. Description of the Related Art**

In spite of all the efforts made by human beings over the past decades, cancer is still one of incurable diseases. Cancer is one of such diseases that threaten human health most seriously, which is caused by unlimited, uncontrollable proliferation and immortalization of cells resulted from a series of mutation. Many biochemical mechanisms involved in cancer have been disclosed, and thereafter various therapeutic agents have been developed. However, none of them are yet a fundamental treatment.

Therefore, it is a continuous request to identify *in vivo* molecules involved in cancer and to develop a novel drug targeting the said molecules. To improve the therapeutic effect, combinations of some of the drugs have been tried. According to the remarkable advancement in the fields of cancer cell biology, medicinal chemistry, etc., many anti-cancer agents have been developed, which are exemplified by Taxol, rapamycin, and 17-allylaminogeldanamycin (17-AAG). In addition, a novel anti-cancer agent, Gleevec, characterized by a novel functional mechanism is now under the development.

Drugs designed to suppress cancer metastasis are largely functioning to inhibit the function of adhesion molecules including integrin family such as vitronectin, laminin, and fibronectin, which are major components of the extracellular matrix, or to inhibit matrix metalloproteinase (MMP) and collagenase IV (Cancer Research 53, 2087-2091, 1993). Major targets of the drugs to suppress cancer metastasis are as follows; Src, focal adhesion kinase, integrin receptor, vascular endothelial growth factor receptor (VEGF receptor), epidermal growth factor receptor (EGF receptor), Her-2/neu, c-Met, Ras/Rac GTPases, Raf kinase, farnesyl diphosphate synthase, and matrix metalloproteases.

Hypoxia-inducible factor-1 (HIF-1) is a transcription factor induced in hypoxia, which is known as the most important molecule for regulating the adaptation of cancer cells to the hypoxic condition. In particular, the level of HIF-$1\alpha$ protein is closely related to the prognosis of cancer patients. HIF-1 is activated by the stimulation of cancer cell growth factor, hypoxic condition, the activation of oncogene, or the inactivation of tumor suppressor gene such as pVHL. The activated HIF-1 induces the expressions of the genes such as hexokinase 2, glucose transporter 1, erythropoietin, IGF-2, endoglin, VEGFA, MMP-2, uPAR, and MDR1, and as a result the resistance against apoptosis, angiogenesis, cell proliferation, cell migration or metastasis, and cell invasion are all increased, leading to the malignance of cancer cells.

Cytochrome b5 reductase 3 (cyb5R3) exists as binding to endoplasmic reticulum membranes, mitochondrial membranes, and other membranes or as dissolved in erythrocytes. Cyb5R3 in erythrocytes is involved in the reduction of methemoglobin. The mutant cyb5R3 gene induces methemoglobinemias, and as a result the concentration of methemoglobin is excessively high in blood so that oxygen delivery is reduced, leading to cyanosis and hypoxia. Cyb5R3 in membranes is composed of membrane-bound domain and active domain, which is known to be involved in the elongation and desaturation of fatty acid, cholesterol biosynthesis, and drug metabolism. Therefore, studies to develop a diagnostic and therapeutic composition using cyb5R3 have been actively undergoing.

As an example of using cyb5R3 in relation to cancer, PCT/NL2007/000112 describes that a sample was separated from a breast cancer patient and the expression of a protein expected to be sensitive to anti-estrogen was investigated in order to measure the sensitivity of anti-estrogen treatment in breast cancer, resulting in the confirmation that cyb5R3 could be useful as a diagnostic kit for the anti-estrogen treatment because cyb5R3 could be used as an index that could predict the tamoxifen-resistance in breast cancer patients. LEE *et al* reported that cyb5R3 called as diaphorase 1 was up-regulated in a lung cancer mouse model transformed with K-*ras* so that cyb5R3 could be used as a marker for cancer diagnosis or as a target molecule to prevent cancer (LEE et al, Int. J. Oncol., 34: 161-172, 2009). However, there has been no report explaining the relation between HIF-$1\alpha$ and cyb5R3 and concerning a pharmaceutical composition for inhibiting cancer using the same.

CN 1 303 926 A describes a polypeptide-NADH-cyb5R1, polynucleotides coding said polypeptide and a method for producing said polypeptide by using DNA recombination techniques. Said document also describes methods for curing several diseases, such as malignant tumor, blood disease, HIV infection, immunological diseases and various inflammations by using said polypeptide. However cyb5R1 and cyb5R3 have different functions in HIF-1 control, they have different locations within the cell and they have very different amino acid sequences.

Thus, the present inventors tried to disclose the cancer inhibiting effect of cyb5R3. As a result, the inventors confirmed that the expression of HIF-$1\alpha$ expression was significantly reduced in the cancer cells particularly over-expressing cyb5R3, resulting in the inhibition of cancer cell growth, *in vivo* tumor growth and metastasis, and further confirmed thereafter that cyb5R3 could be useful as an active ingredient of a pharmaceutical composition for preventing and treating cancer, leading to the completion of the present invention.

**SUMMARY OF THE INVENTION**

It is an object of the present invention to provide a pharmaceutical composition for preventing and treating at least certain cancers, comprising cytochrome b5 reductase 3 (cyb5R3) protein as an active ingredient.

To achieve the above object, the present invention provides a pharmaceutical composition for use in preventing and treating one or more cancers selected from the group consisting of colorectal cancer, breast cancer, pancreatic cancer, prostate cancer, lung cancer and kidney cancer, comprising cytochrome b5 reductase 3 (cyb5R3) protein consisting of the amino acid sequence represented by SEQ ID NO: 1 as an active ingredient.

The present disclosure also describes a method for treating said cancers containing the step of administering cyb5R3 protein to a subject in need.

The present disclosure further describes a method for preventing said cancers containing the step of administering cyb5R3 protein to a subject in need.

The present disclosure also describes a use of cyb5R3 protein as an active ingredient of a pharmaceutical composition for preventing and treating said cancers.

The present invention also provides a pharmaceutical composition for use in preventing and treating one or more cancers selected from the group consisting of colorectal cancer, breast cancer, pancreatic cancer, prostate cancer, lung cancer and kidney cancer, comprising the vector containing the polynucleotide encoding cyb5R3 protein consisting of the amino acid sequence represented by SEQ ID NO: 1 or the cell containing the vector as an active ingredient.

The present disclosure also describes a method for treating said cancers containing the step of administering the vector containing the polynucleotide encoding cyb5R3 protein or the cell containing the said vector to a subject in need.

The present disclosure also describes a method for preventing said cancers containing the step of administering the vector containing the polynucleotide encoding cyb5R3 protein or the cell containing the said vector to a subject in need.

The present disclosure also describes a use of the vector containing the polynucleotide encoding cyb5R3 protein or the cell containing the said vector as an active ingredient of a pharmaceutical composition for preventing and treating said cancers.

The present disclosure also describes a health food for preventing and improving said cancers comprising cyb5R3 protein as an active ingredient.

The present invention also provides a method for detecting a protein in order to provide information necessary for the diagnosis of one or more cancers selected from the group consisting of colorectal cancer, breast cancer, pancreatic cancer, prostate cancer, lung cancer and kidney cancer, comprising the following steps:

1) measuring the expression of cyb5R3 protein in a sample originated from the test subject of the experimental group;
2) comparing the cyb5R3 protein expression measured in step 1) with that of the normal sample of the control; and
3) determining a risk of cancer outbreak by the decrease of the cyb5R3 protein expression, compared with that of the control.

In addition, the present invention provides a method for screening an anticancer agent candidate for one or more cancers selected from the group consisting of colorectal cancer, breast cancer, pancreatic cancer, prostate cancer, lung cancer and kidney cancer, comprising the following steps:

1) treating a test compound or a composition to the cell line expressing cyb5R3 protein consisting of the amino acid sequence represented by SEQ ID NO: 1;
2) measuring the cyb5R3 protein expression in the cell line treated in step 1); and
3) selecting a test compound or a composition that could increase the cyb5R3 protein expression in the cell line of step 2), compared with the control cell line non-treated with the test compound or the composition.

**ADVANTAGEOUS EFFECT**

In the cancer cells over-expressing cytochrome b5 reductase 3 (cyb5R3), the expression of hypoxia-inducible factor-1 (HIF-1) was significantly reduced to inhibit the cancer cell growth and to inhibit *in vivo* tumor growth and metastasis. Therefore, the cyb5R3 gene or protein of the present invention could be efficiently used as an active ingredient of a pharmaceutical composition for preventing and treating cancer.

**BRIEF DESCRIPTION OF THE DRAWINGS**

The application of the preferred embodiments of the present invention is best understood with reference to the accompanying drawings, wherein:

Figure 1 is a diagram illustrating the expression of cytochrome b5 reductase 3 (cyb5R3) protein in various cancer cell lines.
Figure 2 is a diagram illustrating the expression of cyb5R3 in the colon cancer tissue.
Figure 3 is a diagram illustrating the expression of hypoxia-inducible factor-1 (HIF-1) in the cancer cell line according to the inhibition of cyb5R3.
Figure 4 is a set of diagrams illustrating the expression of HIF-1 in the cancer cell line according to the over-expression of cyb5R3;
Figure 4a presents the map of the vector used for the over-expression of cyb5R3; and
Figure 4b presents the expressions of cyb5R3 and HIF-1 in the cancer cell line over-expressing cyb5R3.
Figure 5 is a set of diagrams illustrating the oxygen consumption rate (OCR) of mitochondria in the cancer cell line according to the over-expression of cyb5R3;
Figure 5a presents the changes in the basal respiration rate and the maximum respiration rate of the cancer cell line according to the over-expression of cyb5R3; and
Figure 5b presents the decrease of the total respiration rate of the cancer cell line according to the over-expression of cyb5R3.
Figure 6 is a set of diagrams illustrating the cell growth inhibiting effect according to the over-expression of cyb5R3 in the cancer cell line;
Figure 6a presents the over-expression of cyb5R3 in HCT116 cell line according to the amount of the vector DNA used in order to induce the over-expression of cyb5R3; and
Figure 6b presents the cell growth inhibiting effect shown in the cancer cell line according to the amount of the vector DNA used in order to induce the over-expression of cyb5R3.
Figure 7 is a diagram illustrating the comparison of the expressions of cyb5R3 and HIF-1 protein in the cell line over-expressing cyb5R3 under the normal condition and hypoxic condition.
Figure 8 is a diagram illustrating the tumor growth inhibiting effect of cyb5R3 in the mouse transplanted with a tumor.
Figure 9 is a set of diagrams illustrating the tumor formation inhibiting effect of cyb5R3 in the mouse transplanted with a tumor;
Figure 9a presents the tumor growth over the time in the mouse transplanted with a tumor; and
Figure 9b presents the weight of the tumor 17 days after the tumor transplantation.

**DESCRIPTION OF THE PREFERRED EMBODIMENTS**

Hereinafter, the present invention is described in detail.
The present invention provides a pharmaceutical composition for use in preventing and treating one or more cancers selected from the group consisting of colorectal cancer, breast cancer, pancreatic cancer, prostate cancer, lung cancer and kidney cancer comprising cytochrome b5 reductase 3 (cyb5R3) protein consisting of the amino acid sequence represented by SEQ ID NO: 1 as an active ingredient.
The present disclosure also describes a method for treating said cancers containing the step of administering cyb5R3 protein to a subject in need.
The present disclosure further describes a method for preventing said cancers containing the step of administering cyb5R3 protein to a subject in need.
The present disclosure also describes a use of cyb5R3 protein as an active ingredient of a pharmaceutical composition for preventing and treating said cancers.
The present invention also provides a pharmaceutical composition for use in preventing and treating one or more cancers selected from the group consisting of colorectal cancer, breast cancer, pancreatic cancer, prostate cancer, lung cancer and kidney cancer, comprising the vector containing the polynucleotide encoding cyb5R3 protein consisting of the amino acid sequence represented by SEQ ID NO: 1 or the cell containing the vector as an active ingredient.
The present disclosure also describes a method for treating said cancers containing the step of administering the vector containing the polynucleotide encoding cyb5R3 protein or the cell containing the said vector to a subject in need.
The present disclosure also describes a method for preventing said cancers containing the step of administering the vector containing the polynucleotide encoding cyb5R3 protein or the cell containing the said vector to a subject in need.
The present disclosure also describes a use of the vector containing the polynucleotide encoding cyb5R3 protein or the cell containing the said vector as an active ingredient of a pharmaceutical composition for preventing and treating said cancers.

The cyb5R3 protein fragment useable in the invention consists of the amino acid sequence represented by SEQ ID NO: 1.

The cyb5R3 protein herein preferably inhibits hypoxia-inducible factor-1 (HIF-1) in cancer cells.

The cancer herein is one or more cancers selected from the group consisting of colorectal cancer, breast cancer, pancreatic cancer, prostate cancer, lung cancer, and kidney cancer, and preferably colorectal cancer.

The vector herein is preferably the vector containing a linear DNA expressed in human or animal cells, a plasmid vector, a vector containing the viral expression vector, or a recombinant virus vector including a recombinant retrovirus vector, a recombinant adenovirus vector, a recombinant adeno-associated virus (AAV) vector, a recombinant herpes simplex virus vector, or a recombinant lentivirus vector.

The cell herein is preferably selected from the group consisting of hematopoietic stem cells, dendritic cells, autologous tumor cells, and established tumor cells.

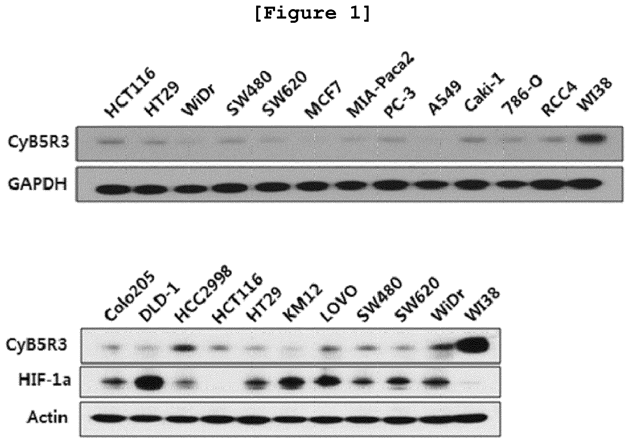

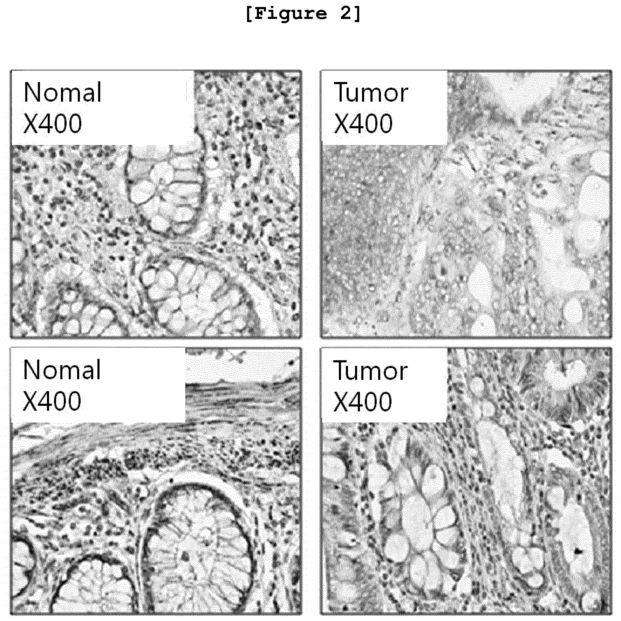

In a preferred aspect, the expressions of cyb5R3 and HIF-1 in a cancer cell line were investigated. As a result, the cyb5R3 expression was significantly reduced in the cancer cell line and the cancer tissues, compared with in the normal cells. In the meantime, the HIF-1$\alpha$ expression was significantly increased, suggesting that the HIF-1$\alpha$ had the inverse correlation with the cyb5R3 (see Figures 1 and 2).

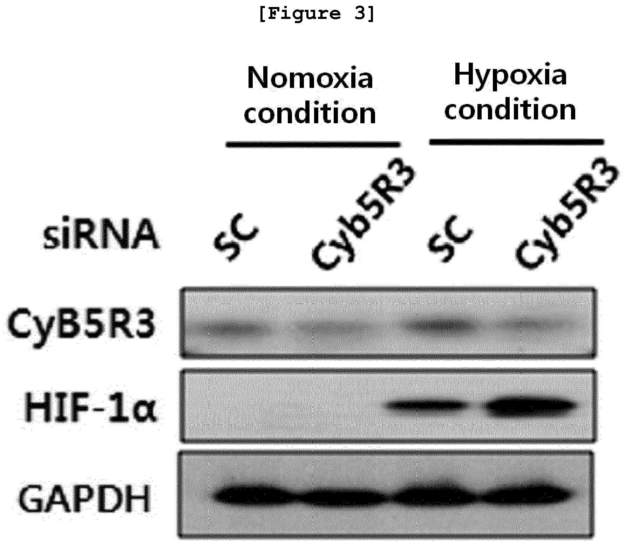

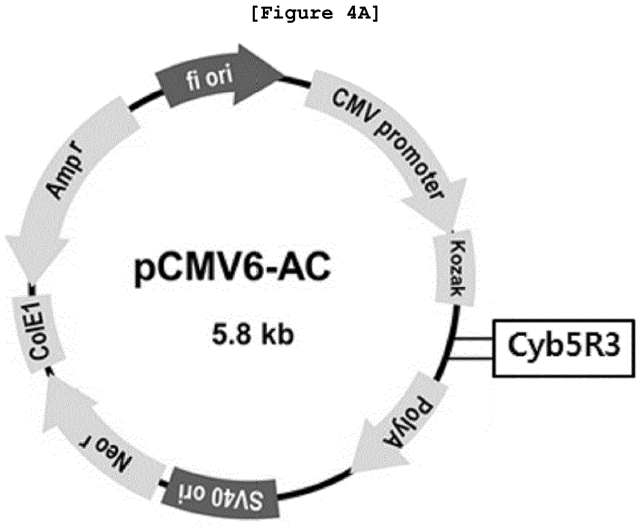

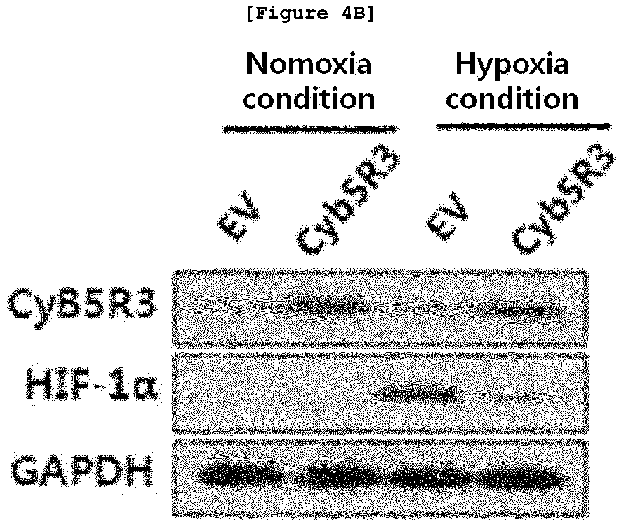

The present inventors also investigated the expression of HIF-1 protein in the cancer cell line wherein cyb5R3 protein was down-regulated or up-regulated. As a result, when HIF-1$\alpha$ was induced under hypoxic condition, HIF-1$\alpha$ was expressed in the cell line wherein the cyb5R3 expression was suppressed (that is cyb5R3 was not expressed). In the meantime, the expression of HIF-1$\alpha$ in the cell line over-expressing cyb5R3 was inhibited (see Figures 3 and 4).

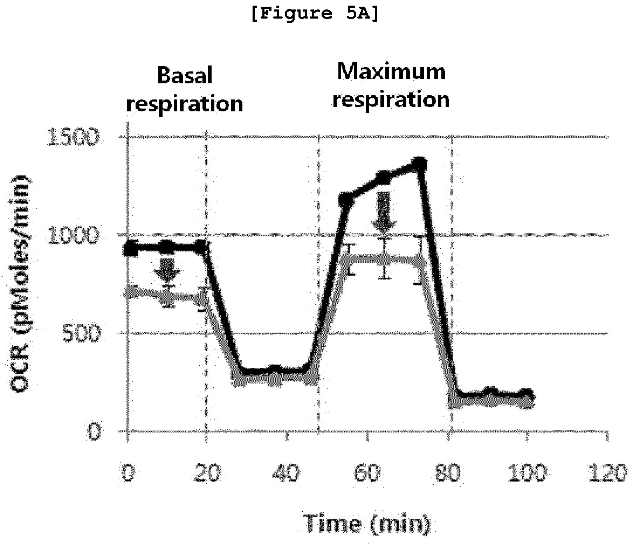

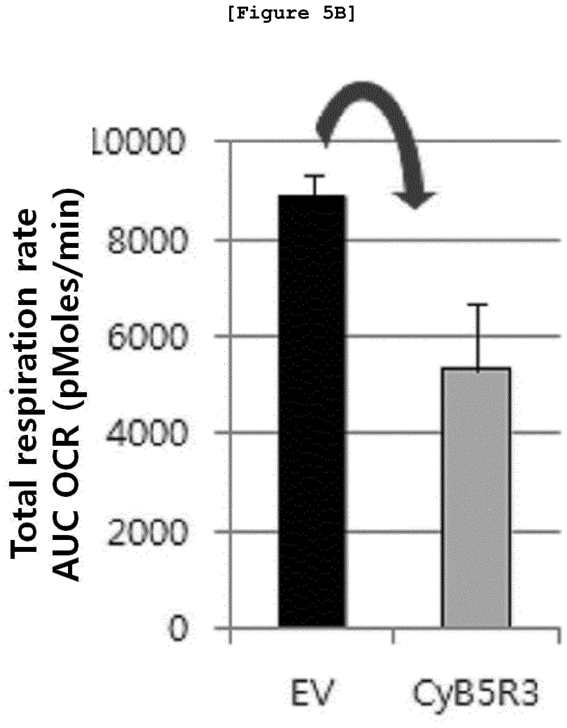

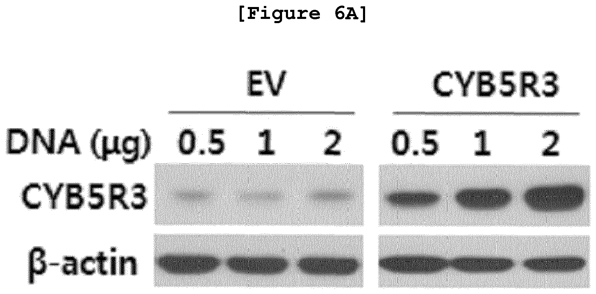

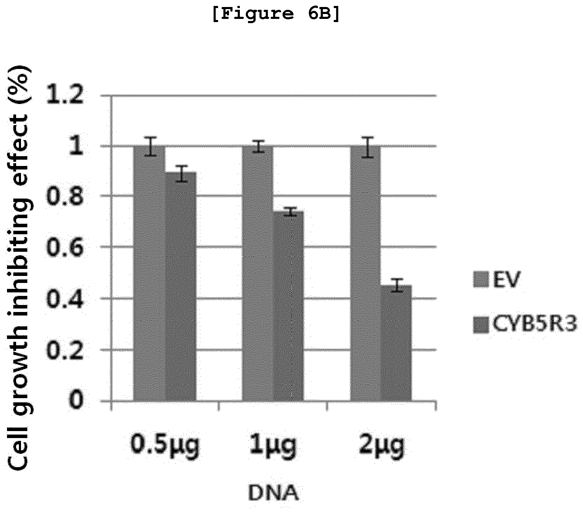

The present inventors investigated the inhibitory effect of cyb5R3 on the cancer cell growth. As a result, it was confirmed that the oxygen consumption rate (OCR) of mitochondria and the cancer cell growth were significantly inhibited in the cancer cell line over-expressing cyb5R3 (see Figures 5 and 6).

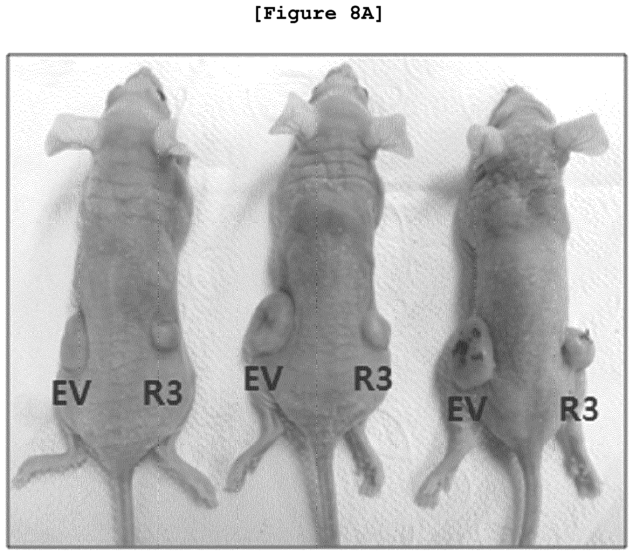

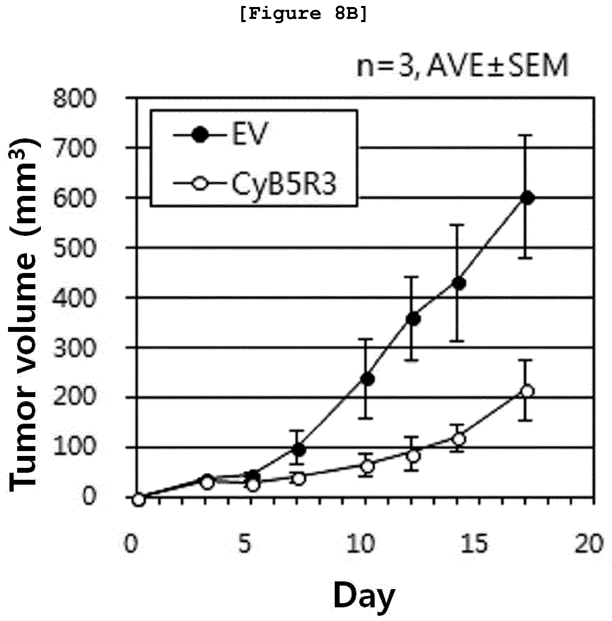

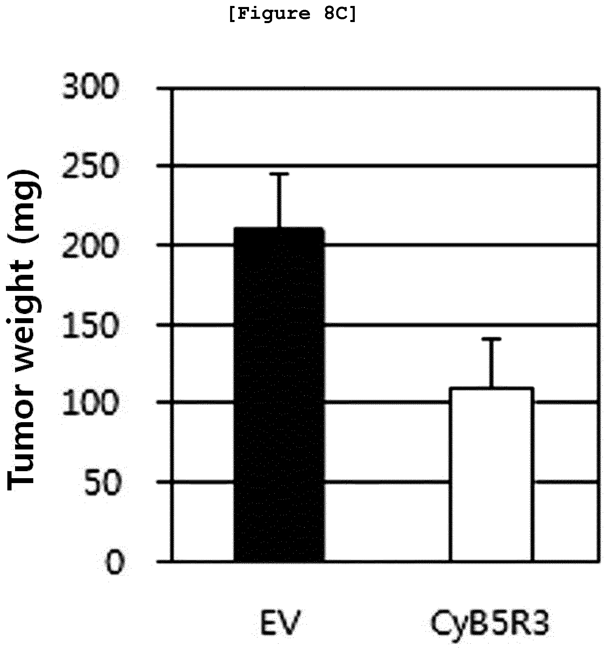

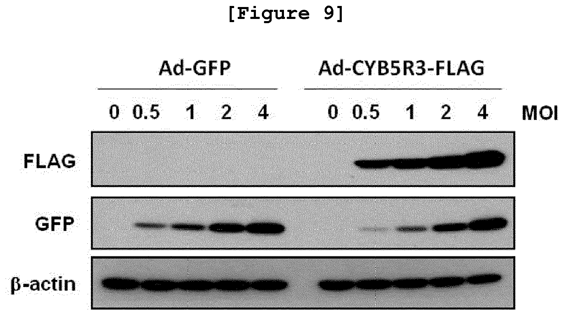

The present inventors also investigated the inhibitory effect of cyb5R3 on the cancer cell growth *in vivo.* As a result, it was confirmed that the tumor size and weight were effectively reduced when the cell line over-expressing cyb5R3 was inserted in the mouse transplanted with a tumor (see Tables 2 and 3, and Figures 8 and 9).

In conclusion, in the cancer cell line over-expressing cyb5R3, the expression of HIF-1$\alpha$ was significantly reduced, resulting in the suppression of cancer cell growth and in the inhibition of *in vivo* tumor growth and metastasis. Therefore, the cyb5R3 gene or protein can be efficiently used as an active ingredient of a pharmaceutical composition for use in preventing and treating certain cancers.

The pharmaceutically effective dosage of the composition of the present invention can be determined by considering various factors such as administration method, target area, patient condition, etc. Thus, the dosage for human body has to be determined with the consideration of safety and efficiency at the same time. It is also possible to predict the effective dosage based on the effective dosage confirmed by animal test. Various factors that have to be considered for the determination of the effective dosage are described in the following articles: Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The composition for use of the present invention can include any generally used carrier, diluent, excipient, or a combination of at least two of those. The pharmaceutically acceptable carrier can be any carrier that is able to deliver the composition for use of the present invention in human body without limitation, which is exemplified by the compounds described in Merck Index, 13th ed., Merck & Co. Inc., such as saline, sterilized water, Ringer's solution, buffered saline, dextrose solution, maltodextrin solution, glycerol, ethanol, and a mixture comprising one or more of those components. If necessary, a general additive such as antioxidant, buffer, and bacteriostatic agent can be additionally added. The composition of the present invention can be formulated in different forms including aqueous solutions, suspensions and emulsions for injection, pills, capsules, granules or tablets by mixing with diluents, dispersing agents, surfactants, binders and lubricants. The composition can further be prepared in suitable forms for each disease or according to ingredients by following the method represented in Remington's Pharmaceutical Science (Mack Publishing Company, Easton PA, 18th, 1990).

The composition for use of the present invention can additionally include, in addition to the active ingredient, one or more effective ingredients having the same or similar effect with the same. The composition for use of the present invention includes the protein fragments preferably by 0.0001 ~ 10 weight%, and more preferably by 0.001 ~ 1 weight% for the total weight of the composition.

The composition for use of the present invention can be administered orally or parenterally (for example, intravenous, hypodermic, peritoneal or local injection). The effective dosage of the composition can be determined according to weight, age, gender, health condition, diet, administration frequency, administration method, excretion and severity of a disease. The dosage is 0.0001 ~ 10 mg/ml per day and preferably 0.0001 ~ 5 mg/ml per day, and administration frequency is once a day or preferably a few times a day.

The composition for use of the present invention contains preferably 0.05 ~ 500 mg of the vector containing the polynucleotide encoding cyb5R3, and more preferably 0.1 ~ 300 mg of the same. The composition for use of the present invention contains preferably $10^3 \sim 10^{12}$ IU ($10 \sim 10^{10}$ PFU) of the recombinant virus containing the polynucleotide encoding C12orf59 originated peptide, and more preferably $10^5 \sim 10^{10}$ IU of the same.

The composition for use of the present invention contains preferably $10^3 \sim 10^8$ of the cells containing the polynucleotide encoding cyb5R3 protein, and more preferably $10^4 \sim 10^7 \sim$ of the same.

In the composition for use comprising the vector containing the polynucleotide encoding cyb5R3 protein or the cell containing the vector, the effective dosage of the vector is $0.05 \sim 12.5$ mg/kg and preferably $0.1 \sim 10$ mg/kg, the effective dosage of the recombinant virus vector is $10^7 \sim 10^{11}$ virus particles ($10^5 \sim 10^9$ IU)/kg and preferably $10^8 \sim 10^{10}$ virus particles ($10^6 \sim 10^8$ IU)/kg, and the effective dosage of the cell is $10^3 \sim 10^6$ cells/kg and preferably $10^2 \sim 10^5$ cells/kg. The composition can be administered $2 \sim 3$ times a day. The dosage and composition are not limited to the above, and can be adjusted according to the patient's condition and the severity of nerve disorder.

The present disclosure describes a health food for preventing and improving said cancers which comprises cyb5R3 protein as an active ingredient.

In the cancer cell line over-expressing cyb5R3, the expression of HIF-1α was significantly reduced, resulting in the suppression of cancer cell growth and in the inhibition of *in vivo* tumor growth and metastasis. Therefore, the cyb5R3 protein useable in the present invention can be efficiently used as an active ingredient of a health food for preventing and improving cancer

The food herein is not limited. For example, the composition for use of the present invention can be added to meats, sausages, breads, chocolates, candies, snacks, cookies, pizza, ramyuns, flour products, gums, dairy products including ice cream, soups, beverages, tea, drinks, alcohol drinks and vitamin complex, etc, and in a wide sense, almost every food applicable in the production of health food can be included.

The cyb5R3 protein useable in the present invention can be used as a food additive. In that case, the cyb5R3 protein can be added as it is or as mixed with other food components according to the conventional method. The mixing ratio of active ingredients can be regulated according to the purpose of use (prevention or improvement). In general, to produce health food or beverages, the cyb5R3 protein is added preferably by $0.1 \sim 90$ weight part. However, if long term administration is required for health and hygiene or regulating health condition, the content can be lower than the above but higher content can be accepted as well since the cyb5R3 protein has been proved to be very safe.

The health beverages containing the composition for use of the present invention can additionally include various flavors or natural carbohydrates, etc, like other beverages. The natural carbohydrates above can be one of monosaccharides such as glucose and fructose; disaccharides such as maltose and sucrose, polysaccharides such as dextrin and cyclodextrin; and sugar alcohols such as xilytole, sorbitol and erythritol. Besides, natural sweetening agents (thaumatin, stevia extract, for example rebaudioside A, glycyrrhizin, etc.) and synthetic sweetening agents (saccharin, aspartame, etc.) can be included as a sweetening agent. The content of the natural carbohydrate is preferably $1 \sim 20$ g and more preferably $5 \sim 12$ g in 100 ml of the composition.

In addition to the ingredients mentioned above, the cyb5R3 protein can include in variety of nutrients, vitamins, minerals (electrolytes), flavors including natural flavors and synthetic flavors, coloring agents and extenders (cheese, chocolate, etc.), pectic acid and its salts, alginic acid and its salts, organic acid, protective colloidal viscosifiers, pH regulators, stabilizers, antiseptics, glycerin, alcohols, and carbonators which used to be added to soda, etc. The cyb5R3 protein can also include natural fruit juice, fruit beverages and/or fruit flesh addable to vegetable beverages. All the mentioned ingredients can be added singly or together. The mixing ratio of those ingredients does not matter in fact, but in general, each can be added by $0.1 \sim 20$ weight part per 100 weight part of the cyb5R3 protein useable in the invention.

The present invention also provides a method for detecting a protein in order to provide information necessary for the diagnosis of one or more cancers selected from the group consisting of colorectal cancer, breast cancer, pancreatic cancer, prostate cancer, lung cancer and kidney cancer, comprising the following steps:

> 1) measuring the expression of cyb5R3 protein consisting of the amino acid sequence represented by SEQ ID NO: 1 in a sample originated from the test subject of the experimental group;
> 2) comparing the cyb5R3 protein expression measured in step 1) with that of the normal sample of the control; and
> 3) determining a risk of cancer by the decrease of the cyb5R3 protein expression, compared with that of the control.

The expression of cyb5R3 protein in step 1) is preferably measured by one of the detection methods selected from the group consisting of Western blotting, enzyme-linked immunosorbent assay (ELISA), immunohistochemical staining, immunoprecipitation, and immunofluorescence.

In addition, the present invention provides a method for screening an anticancer agent candidate for one or more cancers selected from the group consisting of colorectal cancer, breast cancer, pancreatic cancer, prostate cancer, lung cancer and kidney cancer, comprising the following steps:

> 1) treating a test compound or a composition to the cell line expressing cyb5R3 protein consisting of the amino acid sequence represented by SEQ ID NO: 1;

2) measuring the cyb5R3 protein expression in the cell line treated in step 1); and

3) selecting a test compound or a composition that could increase the cyb5R3 protein expression in the cell line of step 2), compared with the control cell line non-treated with the test compound or the composition.

The cyb5R3 is significantly down-regulated in the cancer cell line or cancer tissues, compared with in the normal cell line or normal tissues. In the meantime, the HIF-1$\alpha$ expression is significantly reduced in the cell line over-expressing cyb5R3. Therefore, the cyb5R3 protein can be efficiently used for the method for screening an anticancer agent candidate of the invention and for the method to provide information necessary for the diagnosis of cancer.

Practical and presently preferred embodiments of the present invention are illustrative as shown in the following Examples.

However, it will be appreciated that those skilled in the art, on consideration of this disclosure, may make modifications within the scope of the claims.

Example 1: Expressions of cytochrome b5 reductase 3 (cyb5R3) and hypoxia-inducible factor-1 (HIF-1) in cancer cell lines

<1-1> Cancer cell culture and induction of HIF-1 accumulation

Various cancer cell lines were cultured to accomplish examples of the invention.

Particularly, various human cancer cell lines, shown in Table 1 below, were purchased from ATCC (American Type Culture Collection). RPMI1640 supplemented with 5% fetal bovine serum (FBS) was loaded in a cell culture vessel, to which the cultured cells were inoculated at the density of $5 \times 10^5$ cells/ml, followed by culture in a 37°C 5% $CO_2$ incubator for 24 hours. To induce the accumulation of HIF-1, the cells were further cultured for 12 more hours under hypoxic condition (1% oxygen, 94% nitrogen, and 5% $CO_2$).

[Table 1]

| Various cancer cell lines | | |
|---|---|---|
| Origin | Name | ATCC No. |
| Colorectal cancer cell line | DLD-1 | CCL-221 |
| | HCC2998 | CCL-119 |
| | HCT116 | CCL-247 |
| | KM12 | |
| | LoVo | CCL-229 |
| | SW620 | CCL-227 |
| | WiDr | CCL-218 |
| Breast cancer cell line | MCF-7 | HTB-22 |
| Pancreatic cancer cell line | MIA-Paca2 | CRL-1420 |
| Prostate cancer cell line | PC-3 | CRL-1435 |
| Lung cancer cell line | A549 | CCL-185 |
| Kidney cancer cell line | Caki-1 | HTB-46 |
| | 786-0 | CRL-1932 |
| | RCC-4 | |
| Normal cell line | WI-38 | |

<1-2> Expressions of cyb5R3 and HIF-1 in the HIF-1 induced cancer cell line

To confirm the expressions of HIF-1 and cyb5R3 in the HIF-1 induced cancer cell line, a cell extract was obtained from the cancer cell line, followed by Western blotting.

Particularly, the HIF-1 accumulation induced various cancer cell lines were obtained in Example <1-1>. Each cell line was lysed by using RIPA buffer (radioimmuno precipitation assay buffer; Cell Signaling Technology, USA) and as a result each cell extract was obtained. 30 $\mu$g of the obtained each cell extract was loaded on SDS-PAGE (sodium dodecyl sulfate-polyacrylamide gel electrophoresis) and then transferred on polyvinylidene fluoride membrane. The membrane was reacted with cyb5R3 antibody (Santa Cruz Biotechnology, USA) and HRP-labeled secondary antibody (Amersham-Pharmacia, USA) stepwise to confirm the expression of cyb5R3. The expression of HIF-1$\alpha$ was also confirmed by using HIF-1$\alpha$ antibody (R, D Systems) and HRP-labeled secondary antibody by the same manner as described above. For the control, the expression of GAPDH (glyceradehyde 3- phosphate dehydrogenase) or actin was measured by using rabbit polyclonal GAPDH antibody (Ab Frontier, Korea) or rabbit polyclonal $\beta$-actin antibody (Cell Signaling Technology, USA) by the same manner as described above.

As a result, as shown in Figure 1, the expression of cyb5R3 was significantly reduced in such cell lines as colorectal cancer, breast cancer, pancreatic cancer, prostate cancer, lung cancer, and kidney cancer cell lines, compared with the normal cell line WI-38. In the meantime, the expression of HIF-1$\alpha$ was significantly increased, suggesting that HIF-1$\alpha$ had the inverse correlation with cyb5R3 (Figure 1).

Example 2: Expressions of cyb5R3 in cancer tissue

To investigate the expression of cyb5R3 protein in cancer tissues, immunohistochemistry staining was performed with the colorectal cancer tissues.

Precisely, the samples for the diagnosis were provided from The Catholic University of Korea Daejeon Saint Mary Hospital (Korea), which were then fixed in formalin to prepare paraffin blocks of 20 colorectal cancer tissues. Those paraffin blocks were treated with xylene to eliminate paraffin from the paraffin section. The blocks were heated at 100 °C to recover the antigen, followed by washing with phosphate buffered saline (PBS) three times. The anti-cyb5R3 antibody (Sigma-Aldrich, USA) diluted with the antibody dilution buffer (Covance, USA) at the ratio of 1:200 was used as the primary antibody and the biotin-conjugated antibody was used as the secondary antibody to measure the level of cyb5R3 by using Streptavidin Biotin Universal Detection System (Immunotech, Finland) according to the manufacturer's protocol. Color development was induced with AEC Chromogen Kit (Immunotech, Finland). For the positive control, immunohistostaining was performed with the normal tissue WI-38 by the same manner as described above and the color strength was compared.

As a result, as shown in Figure 2, the cells comprising cyb5R3 were located in the glandular structures in the normal tissues, while the cells expressing cyb5R3 were significantly reduced and the distorted glands were observed at the same time in the colorectal cancer tissues (Figure 2).

Example 3: Expression of HIF-1 according to the expression of cyb5R3 in cancer cell lines

<3-1> Expression of HIF-1 in the cyb5R3 suppressed cancer cell line

To investigate the interrelation between cyb5R3 and HIF-1 expression, the expression of HIF-1 protein was measured in the cancer cell line wherein the cyb5R3 expression was suppressed.

Particularly, 20 nM of cyb5R3 siRNA (Thermo Co.; Cat. No: siGENOME human cyb5R3 (1727) siRNA SMART pool) was treated with Dharmafect (thermo Co.), with which the colon cancer cell line HCT116 was transfected. The transfected cell line was cultured in DMEM (Dulbecco's Modified Eagle's Medium) supplemented with 5% FBS for 24 hours. To induce the accumulation of HIF-1, the cell line was cultured under hypoxic condition (1% oxygen, 94% nitrogen, and 5% $CO_2$) for 12 hours. The expressions of cyb5R3 and HIF-1 protein were measured by the same manner as described in Example <1-2>. For the negative control, the transfected cell line was cultured in the presence of 20% oxygen and the expressions of cyb5R3 and HIF-1 protein were measured by the same manner as described above. As the control protein, the rabbit polyclonal GAPDH antibody (Ab Frontier, Korea) was used and the expression of GAPDH (glyceraldehyde 3-phosphate dehydrogenase) was measured by the same manner as described above.

As a result, as shown in Figure 3, the negative control under oxic condition expressed neither cyb5R3 nor HIF-1$\alpha$. When HIF-1$\alpha$ was induced under hypoxic condition, cyb5R3 was not expressed (Figure 3).

<3-2> Expression of HIF-1 in the cancer cell line over-expressing cyb5R3

To investigate the interrelation between cyb5R3 and HIF-1 expression, cyb5R3 was over-expressed in a cancer cell line and the HIF-1 expression therein was measured.

Particularly, pCMV-AC-cyb5R3 (Figure 4a; Origin, USA), the pCMV6-AV vector containing cyb5R3 gene, was amplified. 4 $\mu$g of the said vector and lipofectamine 2000 (Invitrogen, Carlsbad, CA) were loaded in a 60 mm dish containing DMEM supplemented with 5% FBS, to which $4 \times 10^5$ cells of the colorectal cancer cell line HCT116 were inoculated, followed by culture for 24 hours for DNA transfection. Then, in order to induce the accumulation of HIF-1, the cell line was cultured under hypoxic condition (1% oxygen, 94% nitrogen, and 5% $CO_2$) for 12 hours. The expressions of cyb5R3 and HIF-1 protein were measured by the same manner as described in Example <1-2>. For the negative control, the transfected cell line was cultured in the presence of 20% oxygen and the expressions of cyb5R3 and HIF-1 protein were measured by the same manner as described above. As the control protein, the rabbit polyclonal GAPDH antibody (Ab Frontier, Korea) was used and the expression of GAPDH (glyceraldehyde 3-phosphate dehydrogenase) was measured by the same manner as described above. The expressions of cyb5R3 and HIF-1 protein were measured by the same manner as described in Example <2-1>.

As a result, as shown in Figure 4b, as the expression of cyb5R3 increased in the cell line induced with the over-expression of cyb5R3, the expression of HIF-1$\alpha$ was reduced under hypoxic condition (Figure 4b).

Example 4: Inhibitory effect of cyb5R3 on cancer cell growth

<4-1> Oxygen consumption rate (OCR) of mitochondria in the cancer cell line over-expressing cyb5R3

To investigate the cancer cell growth inhibiting effect of cyb5R3, the oxygen consumption rate of mitochondria in the cancer cell line over-expressing cyb5R3 was measured.

Particularly, the colon cancer cell line HCT116 induced with cyb5R3 over-expression was cultured by the same manner as described in Example <3-2>. $1 \times 10^5$ cultured cells were further cultured in XF24 cell culture plate for 2 hours. The medium was replaced with XF measuring medium, followed by culture in a $CO_2$-free incubator for 1 hour. OCR was measured by using XF24 extracellular flux analyzer (Seahorse Bioscience, USA) three times. The ATP synthesis inhibitor, oligomycin, was treated thereto at the concentration of 1 $\mu$M. Then, OCR was measured three times again. The chemical uncoupler, carbonylcyanide $p$-trifluoromethoxyphenylhydrazone, was treated thereto at the concentration of 0.5 $\mu$M, and then OCR was measured three times. The electron transport system inhibitor, rotenone, was treated thereto at the concentration of 1 $\mu$M, and then OCR was measured twice. 1 $\mu$M of antimycin A was treated thereto and OCR was measured twice again.

As a result, as shown in Figure 5, total OCR in the cell line over-expressing cyb5R3 was reduced 40% (Figure 5b). The basal respiration and the maximum respiration were also significantly reduced (Figure 5a).

<4-2> Cell growth inhibition in the cancer cell line over-expressing cyb5R3

To investigate the cancer cell growth inhibiting effect of cyb5R3, the cell growth inhibition rate in the cancer cell line over-expressing cyb5R3 was measured.

Particularly, pCMV-AC-cyb5R3 (Origin, USA), the pCMV6-AV vector containing cyb5R3 gene, was amplified. The colorectal cancer cell line HCT116 was cultured in DMEM supplemented with 0.5 $\mu$g, 1 $\mu$g, or 2 $\mu$g of the said vector and lipofectamine 2000 (Invitrogen, Carlsbad, CA) for 24 hours for DNA transfection. Upon completion of the culture, the HCT116 cell line transfected with DNA was distributed in a 96 well plate at the density of $3 \times 10^3$ cells / 100 $\mu$l, followed by culture in a 37 °C 5% $CO_2$ incubator for 72 hours. The cells were fixed with 4% formaldehyde at room temperature for 1 hour and washed with PBS. Then, the cells were stained with 0.5% methylene blue (50 $\mu$l/well) at room temperature for 1 hour. The cells were washed with distilled water and dried. 100 $\mu$l of 0.06 N HCl was distributed thereto and the cells were well-mixed with it at room temperature for 10 minutes. $OD_{600}$ was measured by using ELISA plate reader to confirm the cell growth.

As a result, as shown in Figure 6, cancer cell growth was suppressed by the pcMV6-AC-cyb5R3 expression vector that had been inserted in HCT116 cell line to induce the over-expression of cyb5R3 dose-dependently (Figures 6a and 6b).

Example 5: Inhibitory effect of cyb5R3 on *in vivo* cancer cell growth

<5-1> Construction of the cell line over-expressing cyb5R3

To prepare the cell line over-expressing cyb5R3, HCT116 cell line over-expressing cyb5R3 stably was constructed.

Particularly, $4 \times 10^5$ HCT116 cells were inoculated in a 60 mm dish containing DMEM supplemented with 5% FBS, followed by culture in a 37 °C 5% $CO_2$ incubator for 24 hours. 4 $\mu$g of pCMV-AV-cyb5R3 (Origin, USA), the PCMV6-AV vector containing cyb5R3 gene, and lipofectamine 2000 (Invitrogen, Carlsbad, CA) were added thereto, followed by culture at 37 °C for 48 hours for DNA transfection. The HCT116 cells were cultured in DMEM (Dulbecco's Modified Eagle's Medium) supplemented with 0.5 mg/ml of G418 (Gibco, USA) for 2 weeks. The cells transfected with the vector for cyb5R3 over-expression were selected. The single colony of those cells was obtained and cultured. The expression of cyb5R3 was investigated by the same manner as described in Example <1-2>. Thereafter, the HCT cell line over-expressing cyb5R3 stably was selected. For the negative control, the expression vector pCMV-AC (Origin, USA) that did not contain cyb5R3 gene was introduced in HCT116 cell line, that is, cyb5R3 knock-out HCT116 was constructed by the same manner as described above.

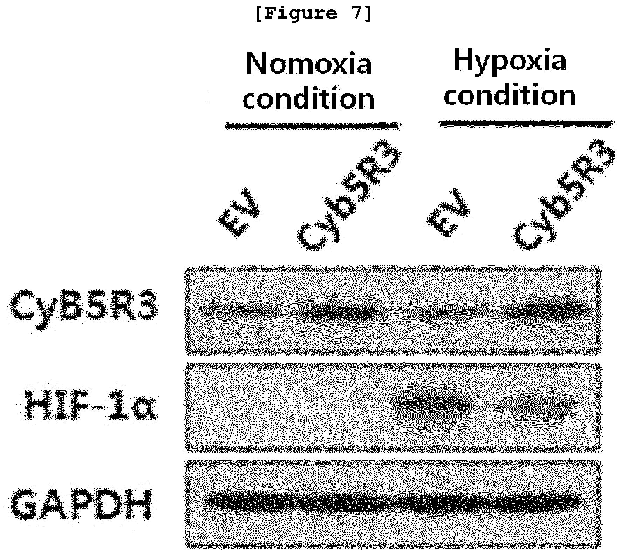

As a result, as shown in Figure 7, the HCT116 cell line over-expressing cyb5R3 stably was collected and the decrease of HIF-1$\alpha$ expression was confirmed in the said HCT116 cell line under hypoxic condition (Figure 7).

<5-2> Inhibitory effect of cyb5R3 on *in vivo* tumor growth

To investigate the *in vivo* cancer cell growth inhibiting effect of cyb5R3, the cell line over-expressing cyb5R3 was inserted *in vivo* and the changes in the size of a tumor therein were confirmed.

Particularly, the HCT116 cell line over-expressing cyb5R3 prepared in Example <5-1> was treated with trypsin and collected. The cells were washed with serum-free medium and diluted at the concentration of $5 \times 10^7$ cells/ml. The diluted cells were injected through the side of 5 female Balb/c specific pathogen free (SPF) nude mice at 6 weeks (SLC-Central Lab. Animal Inc., Korea) via subcutaneous injection to complete the tumor cell transplantation ($1 \times 10^7$ cells / 200 $\mu$l). On day 3, 5, 7, 12, 14, and 17 after the transplantation, the weight of each nude mouse and the size and the growth of tumor were measured by using the mathematical formula 1 of the below. On day 17 after the transplantation, the animals were sacrificed and the weight and the size of tumor were measured to investigate the formation and the growth of tumor. As for the negative control, the cyb5R3 knock-out HCT116 cell line constructed in Example <5-1> was used and the tumor growth was measured by the same manner as described above.

【Mathematical Formula 1】

Tumor Size (mm$^2$) = (length x area x height) / 2

As a result, as shown in Tables 2 and 3, and in Figures 8 and 9, the weight of the mouse was not changed significantly after the transplantation of tumor (Table 2). Compared with the control, in the mouse introduced with the HCT116 cell line over-expressing cyb5R3, the tumor growth was about 65% inhibited (Figure 8 and Table 3). On the 17th day after the tumor transplantation, the weight of the tumor was 11.0$\pm$32.1 mg, which was 48% lower than the tumor weight of the negative control (210.0$\pm$36.1 mg) (Figure 9).

[Table 2]

| Weight changes of the mouse transplanted with the cyb5R3 over-expressing cells and tumor | |
| --- | --- |
| Time (day) | Weight (g) |
| 3 | 19.0$\pm$0.4 |
| 5 | 19.5$\pm$0.4 |
| 7 | 19.5$\pm$0.1 |
| 10 | 18.7$\pm$0.6 |
| 12 | 18.8$\pm$0.3 |
| 14 | 18.6$\pm$0.3 |
| 17 | 18.3$\pm$0.4 |

[Table 3]

| Inhibitory effect of cyb5R3 on *in vivo* tumor growth | | |
| --- | --- | --- |
| Time (day) | Tumor size (mm 3) | |
| | cyb5R3 expression | Negative Control |
| 3 | 32.7$\pm$3.7 | 37.3$\pm$2.0 |
| 5 | 27.0$\pm$7.0 | 46.0$\pm$4.6 |
| 7 | 38.9$\pm$9.4 | 98.9$\pm$33.2 |

(continued)

| Inhibitory effect of cyb5R3 on *in vivo* tumor growth | | |
|---|---|---|
| Time (day) | Tumor size (mm 3) | |
| | cyb5R3 expression | Negative Control |
| 10 | 65.6±23.0 | 238.9±79.4 |
| 12 | 88.0±31.5 | 360.1±83.4 |
| 14 | 119.0±28.4 | 430.5±117.2 |
| 17 | 216.0±60.1 | 603.2±123.3 |

## Claims

1. A pharmaceutical composition for use in preventing and treating one or more cancers selected from the group consisting of colorectal cancer, breast cancer, pancreatic cancer, prostate cancer, lung cancer and kidney cancer, comprising cytochrome b5 reductase 3 (cyb5R3) protein consisting of the amino acid sequence represented by SEQ ID NO: 1 as an active ingredient.

2. A pharmaceutical composition for use in preventing and treating one or more cancers selected from the group consisting of colorectal cancer, breast cancer, pancreatic cancer, prostate cancer, lung cancer and kidney cancer, comprising a vector containing the polynucleotide encoding cyb5R3 protein consisting of the amino acid sequence represented by SEQ ID NO: 1 or the cell containing the vector as an active ingredient.

3. The pharmaceutical composition for use in preventing and treating one or more cancers selected from the group consisting of colorectal cancer, breast cancer, pancreatic cancer, prostate cancer, lung cancer and kidney cancer according to claim 1 or claim 2, wherein the cyb5R3 protein inhibits hypoxia-inducible factor 1 (HIF-1) in cancer cells.

4. The pharmaceutical composition for use in preventing and treating one or more cancers selected from the group consisting of colorectal cancer, breast cancer, pancreatic cancer, prostate cancer, lung cancer and kidney cancer according to claim 2, wherein the vector is a recombinant virus vector and the recombinant virus is selected from the group consisting of retrovirus, adenovirus, adeno-associated virus, herpes simplex virus, and lentivirus.

5. The pharmaceutical composition for use in preventing and treating one or more cancers selected from the group consisting of colorectal cancer, breast cancer, pancreatic cancer, prostate cancer, lung cancer and kidney cancer according to claim 2, wherein the cell is selected from the group consisting of hematopoietic stem cells, dendritic cells, autologous tumor cells, and established tumor cells.

6. A method for detecting a protein to provide information necessary for the diagnosis of one or more cancers selected from the group consisting of colorectal cancer, breast cancer, pancreatic cancer, prostate cancer, lung cancer and kidney cancer, comprising the following steps:

   1) measuring the expression of cyb5R3 protein consisting of the amino acid sequence represented by SEQ ID NO: 1 in a sample originated from the test subject of the experimental group;
   2) comparing the cyb5R3 protein expression measured in step 1) with that of the normal sample of the control; and
   3) determining a risk of cancer by the decrease of the cyb5R3 protein expression, compared with that of the control.

7. A method for screening an anticancer agent candidate for one or more cancers selected from the group consisting of colorectal cancer, breast cancer, pancreatic cancer, prostate cancer, lung cancer and kidney cancer, comprising the following steps:

   1) treating a test compound or a composition to the cell line expressing cyb5R3 protein consisting of the amino acid sequence represented by SEQ ID NO: 1;
   2) measuring the cyb5R3 protein expression in the cell line treated in step 1); and
   3) selecting a test compound or a composition that could increase the cyb5R3 protein expression in the cell line of step 2), compared with the control cell line non-treated with the test compound or the composition.

**Patentansprüche**

1. Pharmazeutische Zusammensetzung zur Verwendung beim Verhüten und Behandeln einer oder mehrerer Krebserkrankungen, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, wobei sie als Wirkbestandteil das Protein Cytochrom-b5-reductase 3 (cyb5R3) umfasst, das aus der Aminosäuresequenz besteht, welche in der SEQ ID Nr.: 1 dargestellt ist.

2. Pharmazeutische Zusammensetzung zur Verwendung beim Verhüten und Behandeln einer oder mehrerer Krebserkrankungen, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, wobei sie als Wirkbestandteil einen Vektor umfasst, der das Polynukleotid enthält, welches für das Protein cyb5R3 codiert, das aus der Aminosäuresequenz besteht, welche in der SEQ ID Nr.: 1 dargestellt ist, oder die Zelle, welche den Vektor enthält.

3. Pharmazeutische Zusammensetzung zur Verwendung beim Verhüten und Behandeln einer oder mehrerer Krebserkrankungen, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, nach Anspruch 1 oder Anspruch 2, wobei das Protein cyb5R3 bei Krebszellen den Hypoxie-induzierten Faktor 1 (HIF-1) hemmt.

4. Pharmazeutische Zusammensetzung zur Verwendung beim Verhüten und Behandeln einer oder mehrerer Krebserkrankungen, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, nach Anspruch 2, wobei es sich bei dem Vektor um einen rekombinanten Virusvektor handelt und das rekombinante Virus der aus der Gruppe ausgewählt ist, welche aus Retroviren, Adenoviren, adenoassoziierten Viren, Herpes-simplex-Viren und Lentiviren besteht.

**5.** Pharmazeutische Zusammensetzung zur Verwendung beim Verhüten und Behandeln einer oder mehrerer Krebserkrankungen, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, nach Anspruch 2, wobei die Zelle aus der Gruppe ausgewählt ist, welche aus hämatopoetischen Stammzellen, dendritischen Zellen, autologen Tumorzellen und etablierten Tumorzellen besteht.

**6.** Verfahren zum Nachweis eines Proteins, um Informationen zu erhalten, die zur Diagnose einer oder mehrerer Krebserkrankungen erforderlich sind, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, wobei es die folgenden Schritte umfasst:

1) Messen der Expression des Proteins cyb5R3, welches aus der Aminosäuresequenz besteht, die in der SEQ ID Nr.: 1 dargestellt ist, in einer Probe, die aus dem zu prüfenden Zielorganismus der Versuchsgruppe stammt.
2) Vergleichen der Expression des Proteins cyb5R3, wie sie in Schritt 1) gemessen wurde, mit derjenigen der normalen Probe aus dem Kontrollansatz; und
3) Bestimmen eines Krebsrisikos anhand der Abnahme der Expression des cyb5R3-Proteins, verglichen mit derjenigen des Kontrollansatzes.

**7.** Verfahren zum Screenen eines Kandidaten für ein Mittel, das eine oder mehrere Krebserkrankungen bekämpft, die aus der Gruppe ausgewählt sind, welche aus dem kolorektalen Karzinom, Brustkrebs, Bauchspeicheldrüsenkrebs, Prostatakrebs, Lungenkrebs und Nierenkrebs besteht, wobei es die folgenden Schritte umfasst:

1) Behandeln einer Zelllinie, die das Protein cyb5R3 exprimiert, welches aus der Aminosäuresequenz besteht, die in der SEQ ID Nr. 1 dargestellt ist, mit einer Prüfverbindung oder einer Zusammensetzung;
2) Messen der Expression des Proteins cyb5R3 bei der Zelllinie, die in Schritt 1) behandelt wurde; und
3) Auswählen einer Prüfverbindung oder einer Zusammensetzung, welche die Expression des Proteins cyb5R3 bei der Zelllinie des Schrittes 2) erhöhen könnte, verglichen der Kontrollzelllinie, die nicht mit der Prüfverbindung oder der Zusammensetzung behandelt wurde.

## Revendications

**1.** Composition pharmaceutique pour une utilisation dans la prévention et le traitement d'un ou de plusieurs cancers choisis dans le groupe constitué par le cancer colorectal, le cancer du sein, le cancer du pancréas, le cancer de la prostate, le cancer du poumon et le cancer du rein, comprenant une protéine cytochrome b5 réductase 3 (cyb5R3) constituée de la séquence d'acides aminés représentée par SEQ ID NO: 1 en tant que principe actif.

**2.** Composition pharmaceutique pour une utilisation dans la prévention et le traitement d'un ou de plusieurs de cancers choisis dans le groupe constitué par le cancer colorectal, le cancer du sein, le cancer du pancréas, le cancer de la prostate, le cancer du poumon et le cancer du rein, comprenant un vecteur contenant le polynucléotide codant pour la protéine cyb5R3 constituée de la séquence d'acides aminés représentée par SEQ ID NO: 1 ou la cellule contenant le vecteur en tant que principe actif.

**3.** Composition pharmaceutique pour une utilisation dans la prévention et le traitement d'un ou de plusieurs de cancers choisis dans le groupe constitué par le cancer colorectal, le cancer du sein, le cancer du pancréas, le cancer de la prostate, le cancer du poumon et le cancer du rein selon la revendication 1 ou la revendication 2, dans laquelle la protéine cyb5R3 inhibe le facteur 1 inductible par l'hypoxie (HIF-1) dans les cellules cancéreuses.

**4.** Composition pharmaceutique pour une utilisation dans la prévention et le traitement d'un ou de plusieurs de cancers choisis dans le groupe constitué par le cancer colorectal, le cancer du sein, le cancer du pancréas, le cancer de la prostate, le cancer du poumon et le cancer du rein selon la revendication 2, dans laquelle le vecteur est un vecteur de type virus recombinant et le virus recombinant est choisi dans le groupe constitué par un rétrovirus, un adénovirus, un virus adéno-associé, un virus de l'herpès simplex et un lentivirus.

**EP 3 053 601 B1**

**5.** Composition pharmaceutique pour une utilisation dans la prévention et le traitement d'un ou de plusieurs de cancers choisis dans le groupe constitué par le cancer colorectal, le cancer du sein, le cancer du pancréas, le cancer de la prostate, le cancer du poumon et le cancer du rein selon la revendication 2, dans laquelle la cellule est choisie dans le groupe constitué par les cellules souches hématopoïétiques, les cellules dendritiques, des cellules tumorales autologues et des cellules tumorales établies.

**6.** Procédé de détection d'une protéine pour fournir des informations nécessaires au diagnostic d'un ou de plusieurs de cancers choisis dans le groupe constitué par le cancer colorectal, le cancer du sein, le cancer du pancréas, le cancer de la prostate, le cancer du poumon et le cancer du rein, comprenant les étapes suivantes :

1) la mesure de l'expression de la protéine cyb5R3 constituée de la séquence d'acides aminés représentée par SEQ ID NO: 1 dans un échantillon issu du sujet d'essai du groupe expérimental ;
2) la comparaison de l'expression de la protéine cyb5R3 mesurée à l'étape 1) avec celle de l'échantillon normal du témoin ; et
3) la détermination d'un risque de cancer par la diminution de l'expression de la protéine cyb5R3, par rapport à celle du témoin.

**7.** Procédé de criblage d'un agent anticancéreux candidat pour un ou plusieurs cancers choisis dans le groupe constitué par le cancer colorectal, le cancer du sein, le cancer du pancréas, le cancer de la prostate, le cancer du poumon et le cancer du rein, comprenant les étapes suivantes :

1) le traitement d'un composé d'essai ou d'une composition sur la lignée cellulaire exprimant la protéine cyb5R3 constituée de la séquence d'acides aminés représentée par SEQ ID NO: 1 ;
2) la mesure de l'expression de la protéine cyb5R3 dans la lignée cellulaire traitée à l'étape 1) ; et
3) la sélection d'un composé d'essai ou d'une composition qui pourrait augmenter l'expression de la protéine cyb5R3 dans la lignée cellulaire de l'étape 2), par rapport à la lignée cellulaire témoin non traitée avec le composé d'essai ou la composition.

[Figure 1]

EP 3 053 601 B1

[Figure 2]

[Figure 3]

[Figure 4A]

[Figure 4B]

[Figure 5A]

[Figure 5B]

[Figure 6A]

[Figure 6B]

[Figure 7]

[Figure 8A]

[Figure 8B]

[Figure 8C]

[Figure 9]

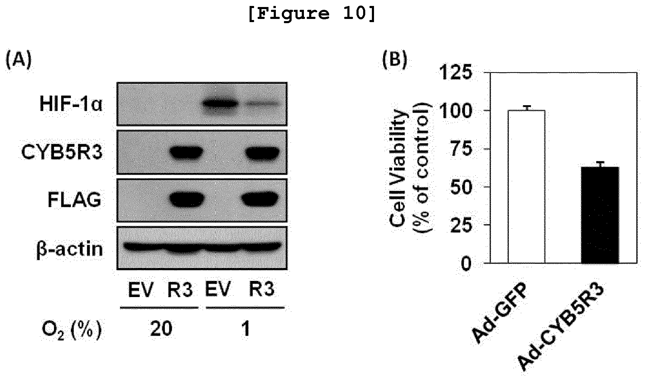

[Figure 10]

(A)

(B)

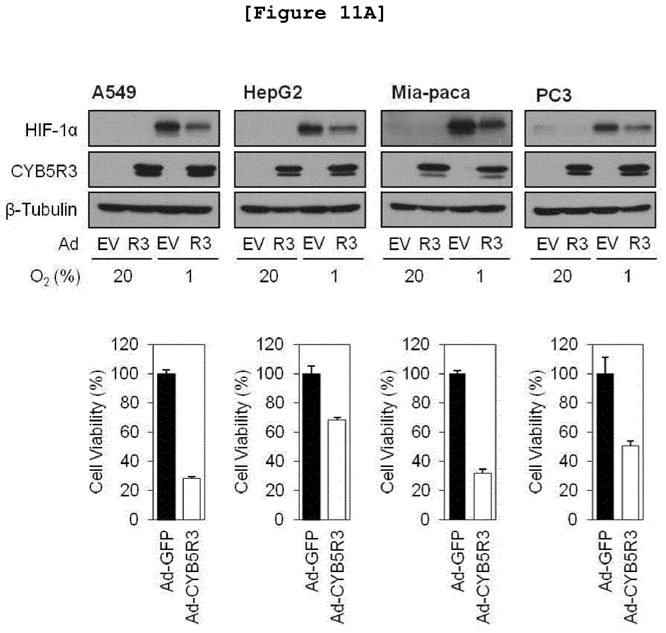

[Figure 11A]

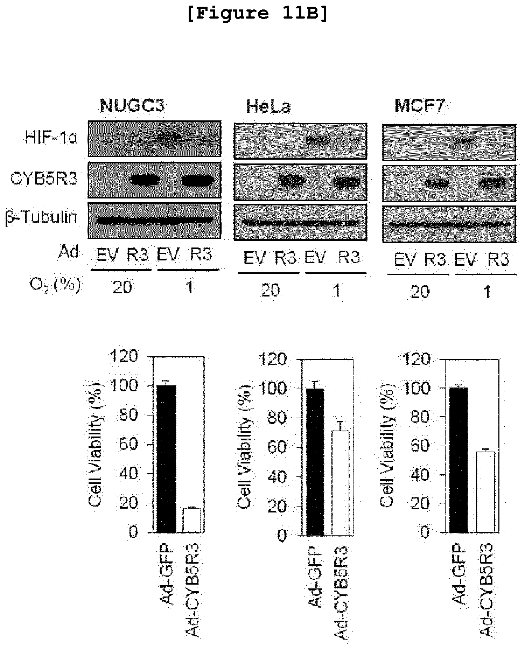

[Figure 11B]

EP 3 053 601 B1

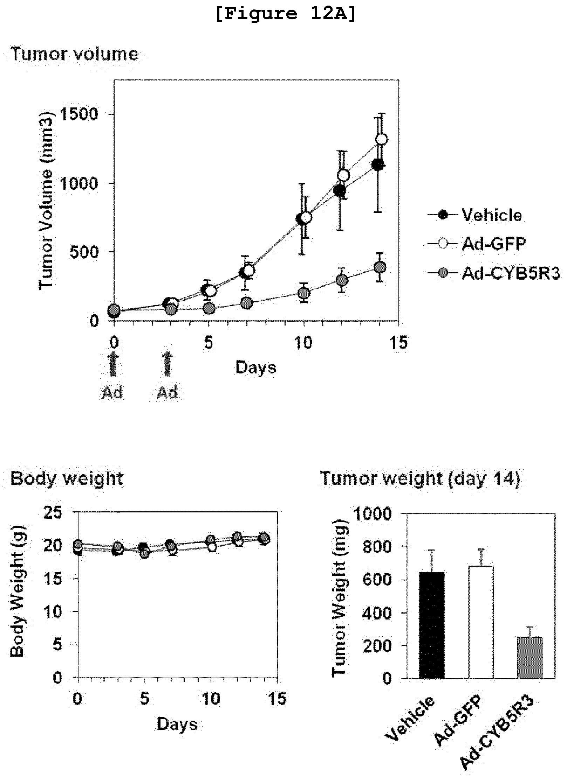

[Figure 12A]

## Tumor volume

- Vehicle
- Ad-GFP
- Ad-CYB5R3

## Body weight

## Tumor weight (day 14)

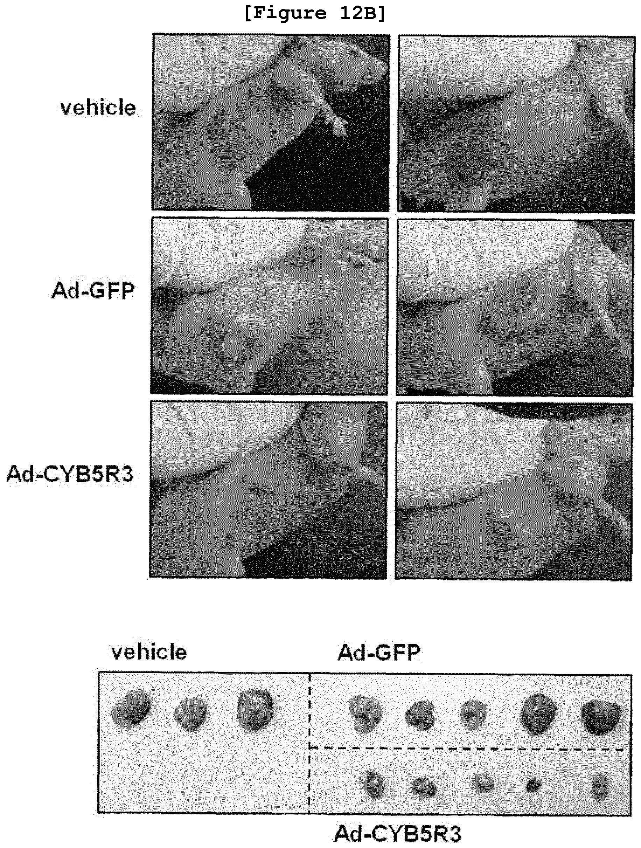

[Figure 12B]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- NL 2007000112 W
- CN 1303926 A
- EP 14851311 A
- KR 1020130117527

### Non-patent literature cited in the description

- *Cancer Research,* 1993, vol. 53, 2087-2091
- **LEE et al.** *Int. J. Oncol.,* 2009, vol. 34, 161-172
- Goodman and Gilman's The Pharmacological Basis of Therapeutics. Pergamon Press, 2001
- Remington's Pharmaceutical Sciences. Mack Publishing Co, 1990
- Merck Index. Merck & Co. Inc,
- Remington's Pharmaceutical Science. Mack Publishing Company, 1990

SEQUENCE LISTING

<110> Korea Research Institute of Bioscience and Biotechnology
WON, MI SUN
BAN, HYUN SEUNG
KIM, BO KYUNG
NAM, SOON WOO
LEE, KYEONG
LEE, YOUNG-JU
LEE, HONG-SUB
KIM, DONG UK
PARK, KI CHEOL
IM, JOO-YOUNG
OH, KYOUNG-JIN

<120> Pharmacological composition containing a cyb5R3 protein or a polynucleotide encoding cyb5R3 for the prevention and treatment of cancer

<130> P-WON-097/WOEP

<140> EP14851311.2
<141> 2014-09-30

<150> KR10-2013-0117527
<151> 2013-10-01

<160> 1

<170> PatentIn version 3.5

<210> 1
<211> 301
<212> PRT
<213> Homo sapiens

<400> 1

```
Met Gly Ala Gln Leu Ser Thr Leu Gly His Met Val Leu Phe Pro Val
1               5                   10                  15

Trp Phe Leu Tyr Ser Leu Leu Met Lys Leu Phe Gln Arg Ser Thr Pro
            20                  25                  30

Ala Ile Thr Leu Glu Ser Pro Asp Ile Lys Tyr Pro Leu Arg Leu Ile
        35                  40                  45

Asp Arg Glu Ile Ile Ser His Asp Thr Arg Arg Phe Arg Phe Ala Leu
    50                  55                  60

Pro Ser Pro Gln His Ile Leu Gly Leu Pro Val Gly Gln His Ile Tyr
65              70                  75                  80

Leu Ser Ala Arg Ile Asp Gly Asn Leu Val Val Arg Pro Tyr Thr Pro
            85                  90                  95

Ile Ser Ser Asp Asp Asp Lys Gly Phe Val Asp Leu Val Ile Lys Val
```

```
                    100                    105                    110

        Tyr Phe Lys Asp Thr His Pro Lys Phe Pro Ala Gly Gly Lys Met Ser
                115                120                125

        Gln Tyr Leu Glu Ser Met Gln Ile Gly Asp Thr Ile Glu Phe Arg Gly
            130                135                140

        Pro Ser Gly Leu Leu Val Tyr Gln Gly Lys Gly Lys Phe Ala Ile Arg
        145                150                155                160

        Pro Asp Lys Lys Ser Asn Pro Ile Ile Arg Thr Val Lys Ser Val Gly
                165                170                175

        Met Ile Ala Gly Gly Thr Gly Ile Thr Pro Met Leu Gln Val Ile Arg
                180                185                190

        Ala Ile Met Lys Asp Pro Asp Asp His Thr Val Cys His Leu Leu Phe
                195                200                205

        Ala Asn Gln Thr Glu Lys Asp Ile Leu Leu Arg Pro Glu Leu Glu Glu
                210                215                220

        Leu Arg Asn Lys His Ser Ala Arg Phe Lys Leu Trp Tyr Thr Leu Asp
        225                230                235                240

        Arg Ala Pro Glu Ala Trp Asp Tyr Gly Gln Gly Phe Val Asn Glu Glu
                        245                250                255

        Met Ile Arg Asp His Leu Pro Pro Pro Glu Glu Glu Pro Leu Val Leu
                260                265                270

        Met Cys Gly Pro Pro Pro Met Ile Gln Tyr Ala Cys Leu Pro Asn Leu
                275                280                285

        Asp His Val Gly His Pro Thr Glu Arg Cys Phe Val Phe
        290                295                300
```